# EUROPÄISCHE PATENTANMELDUNG

(11) **EP 2 462 917 A1**
(43) Veröffentlichungstag der Anmeldung: **13.06.2012**
(21) Anmeldenummer: 10194153.2
(22) Anmeldetag: 08.12.2010
(51) Int. Cl.: A61K 8/27, A61K 8/29, A61K 8/49, A61Q 17/02, A61Q 17/04

(54) **Insektenrepellierende Formulierungen von Sonnenschutzmitteln, ein Verfahren zu deren Herstellung sowie deren Verwendung zum Schutz der menschlichen Haut vor UV-Licht und Insektenstichen**

(71) Anmelder: Saltigo GmbH, 40764 Langenfeld (DE)
(72) Erfinder: Dr. Koch, Burkhard, 50823, Köln (DE); Tombeux, Beate, 51373, Leverkusen (DE)
(74) Vertreter: Pettrich, Klaus-Günter

(57) **Zusammenfassung**

Beschrieben werden insektenrepellierende Formulierungen eines Sonnenschutzmittels, enthaltend das Insektenrepellent 1-sek-Butyloxycarbonyl-2-(2-hydroxyethyl)-piperidin (Saltidin^{®}) und 2,2'-[6-(4-methoxyphenyl)-1,3,5-triazin-2,4-diyl]bis{5-[(2-ethylhexyl)oxy]phenol} und Titandioxid oder Zinkoxid als Sonnenschutzmittel. Ebenfalls beschrieben wird ein Verfahren zur Herstellung dieser Formulierungen sowie deren Verwendung zum Schutz der menschlichen Haut vor UV-Licht und Insektenstichen.

## Beschreibung

Die vorliegende Erfindung betrifft Formulierungen eines Sonnenschutzmittels, welche die insekten- und milbenabwehrende Wirkung eines Insektenrepellents mit einem Sonnschutzfaktor vereinigen. Als insektenrepellierende Komponente wird dabei die Verbindung 1-sek-Butyloxycarbonyl-2-(2-hydroxyethyl)-piperidin eingesetzt (auch als 1-Piperidincarboxylsäure-2-(2-hydroyethyl)-1-methylpropylester bezeichnet; CAS-Nr. 119515-38-7), die die folgende Struktur der Formel (1) aufweist

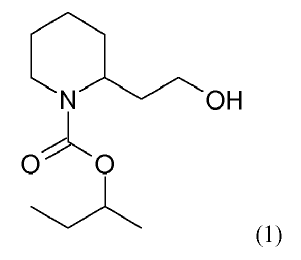

Das Racemat der Verbindung (1) ist unter dem Handelsnamen Saltidin^{®} (andere Namen: Picaridin^{®}, Icaridin^{®}, früher Bayrepel^{®}) bekannt. Weiterhin betrifft die Erfindung ein Verfahren zur Herstellung dieser Formulierungen. Alternativ können anstatt des Racemats auch die vier optischen Isomere von Saltidin^{®} in reiner oder in Kombination mehrerer Isomere als insektenrepellierende Komponente eingesetzt werden. Ein weiterer Gegenstand der Erfindung ist die Verwendung dieser Formulierungen zum Schutz der menschlichen Haut vor UV-Licht und Insektenstichen.

Mittel, die Insekten oder Milben abweisen, werden Insektenrepellents genannt und haben die Aufgabe, schädliche oder lästige Gliederfüßler von Berührung sowie vom Stechen und Saugen oder Beißen an für sie anlockenden Oberflächen, etwa an der Haut von Tieren und Menschen abzuhalten, wenn diese zuvor mit solchen Mitteln behandelt wurden.

Als Repellents wurden bereits zahlreiche Wirkstoffe vorgeschlagen (vergl. z. B. K.H. Büchel in Chemie der Pflanzenschutz- und Schädlingsbekämpfungsmittel; Herausgeber: R. Wegler, Bd. 1, Springer Verlag Berlin, Heidelberg, New York, 1970, S. 487-496).

Besonders bekannt und seit längerer Zeit in Gebrauch sind 3-Methylbenzoesäurediethylamid (DEET), Dimethylphthalat und 2-Ethylhexandiol-1,3, von denen vor allem das DEET in der Praxis eine erhebliche Bedeutung erlangt hat (siehe z.B. R. K. Kocher, R.S. Dixit, C.I. Somaya; Indian J. Med. Res. (1974) 62, 1, 125-133).

Sonnenschutzmittel dienen zur Reduzierung der in die Haut eindringenden UV-Strahlungsmenge und schützen diese so vor den schädlichen Wirkungen wie Sonnenbrand, Hautalterung und im Extremfall Hautkrebs.

Die zeitliche Länge des Sonnenschutzes wird durch den Lichtschutzfaktor (LSF; englisch: SPF = Sun protection factor) ausgedrückt. Dieser gibt an, wie viel länger man sich mit einem Sonnenschutzmittel der Sonne aussetzen kann, ohne einen Sonnenbrand zu erleiden.

Als Sonnenschutzmittel werden sowohl anorganische und organische Verbindungen eingesetzt, die verschiedene Wellenlängen des Sonnenlichtes entweder reflektieren ("Physikalische Filter") oder absorbieren ("Chemische Filter"). Oft werden Kombinationen dieser Mittel eingesetzt, um einen breiten Schutz im UVA- und UVB-Wellenlängenbereich zu erzielen. Als Chemische Filter werden z.B. Derivate von Benzophenon, Campher, Oxybenzon, Trisiloxan, Triazine oder Drometrizol eingesetzt. Als anorganische Filter werden meist Titandioxid- und Zinkoxid-Pigmente verwendet.

Ein weiterer kritischer Punkt bei der Verwendung von Kombinationsmitteln aus Sonnenschutz und Insektenrepellent ist die synergistische Wirkweise in Bezug auf Hautpenetration der aktiven Inhaltsstoffe. In vielen Untersuchungen wurde nachgewiesen, dass z.B. bei der kombinierten Verwendung von Oxybenzon, einem gebräuchlichen UV-Filter, und DEET die Hautpenetration sowohl von DEET als auch von Oxybenzon in synergistischer Weise erhöht wird (z.B. Toxicology and Applied Pharmacology (2007), 223(2), 187-194; Journal of Pharmacy & Pharmaceutical Sciences (2007), 10(1), 17-25; International Journal of Pharmaceutics (2006), 310(1-2), 110-117; British Journal of Dermatology (2005), 152(6), 1263-1267; Can. Journal of Pharmacy and Pharmacology (2004), 56(5), 621-628; Drug Metabolism and Disposition (2004), 32(8), 783-785))

Auch erhöhte sich die Hautpenetration von Schadstoffen, z.B. Herbiziden, wenn DEET in einer Sonnenschutzformulierung verwendet wird (Toxicology and Applied Pharmacology (2004), 195(3), 348-354).

Aus den genannten Gründen wird daher in verschiedenen Publikationen abgeraten, Kombinationsmittel aus Sonnenschutz und Insektenrepellent einzusetzen (z.B. Current opinion in pediatrics (2004), 16(4), 378-84).

Trotz dieser Untersuchungen ist die Kombination von Sonnenschutzmitteln und einem Insektenrepellent wie z.B. DEET Inhalt zahlreicher Patentanmeldungen. Kombinationen von Sonnenschutzmitteln und dem Insektenrepellent Saltidin^{®} sind deutlich weniger oft beschrieben.

Zum Beispiel wird in der WO2007/050653 eine wässrige Formulierung aus einem UV-Filter, Saltidin^{®}, einem Emulgator und einem Filmbildner beschrieben.

In WO2008/024738 wird eine klare, wässrige Formulierung bestehend aus einem hydrophoben UV-Filter, einem Insektenrepellent wie z.B. Saltidin^{®} oder DEET und einem Alkohol mit zwei bis zehn Kohlenstoffatomen beschrieben.

In der WO2005/062860 werden Sonnenschutzformulierungen mit Zusatz von 2-substituierten 3,3-Diaryl-2-propensäurederivaten zur Erhöhung der Stabilität beschrieben. Optional können diese Formulierungen insektenrepellierende Wirkstoffe wie z.B. DEET oder Saltidin^{®} enthalten.

In der WO2003/051319 werden Sonnenschutzformulierungen mit Zusatz von Kaempferia Galanga-Extrakten zur Erhöhung der Stabilität beschrieben. Bevorzugte sonnenschutzaktive Substanzen sind Dibenzoylmethan und Derivate davon. Optional können diesen Formulierungen Insektenrepellents wie DEET oder Saltidin^{®} zugesetzt werden.

In der WO2003/020232 wird eine Formulierung mit einem Insektenrepellent (DEET, Dimethylphthalat, 3-[N-n-butyl-N-acetyl]aminopropionsäureethylester = IR3535), einem oder mehreren UV-Filtern sowie mindestens einem Dialkylnaphthalat beschrieben.

In den genannten Patentanmeldungen finden sich kaum Belege für die Höhe des Sonnenschutzes oder für die Dauer der repellierenden Wirkung. Generell finden sich keine Angaben zu möglicher Hautpenetration von UV-Filter und/oder Repellent. Aufgrund der oben zitierten Studien ist aber davon auszugehen, dass mit Hautpenetration der Wirkstoffe und damit möglichen Gesundheitsgefahren zu rechnen ist.

Der Erfindung lag die Aufgabe zu Grunde, eine Formulierung zu finden, welche breiten UVA/UVB-Schutz mit lang anhaltender repellierender Wirkung gegenüber Insekten kombiniert. Das Produkt zudem soll auch nach Kontakt mit Wasser, z.B. nach dem Schwimmen noch ausreichenden Sonnenschutz bieten. Die Nachteile bekannter kombinierter Formulierungen aus Insektenrepellent und UV-Filter (Hautpenetration, Verminderung des Lichtschutzfaktors durch Zusatz von Insektenrepellent) sollen vermieden werden. Weiterhin soll sich die Formulierung durch ein geringes allergenes oder toxikologisches Potential, z.B. durch Vermeidung von Antioxidantien und Konservierungsmitteln auszeichnen.

Es wurde gefunden, dass durch Kombination spezieller UV-Filter in Verbindung mit dem Insektenrepellent Saltidin^{®} Kombinationsprodukte erhalten werden können, welche lang anhaltende repellierende Wirkung gegenüber Insekten (>7h) mit einem breiten UV-Schutz vereinen.

Gegenstand der vorliegenden Erfindung sind daher insektenrepellierende Formulierungen, enthaltend das Insektenrepellent 1-sek-Butyloxycarbonyl-2-(2-hydroxyethyl)-piperidin und 2,2'-[6-(4-methoxyphenyl)-1,3,5-triazin-2,4-diyl]bis{5-[(2-ethylhexyl)oxy]phenol} und Titandioxid oder Zinkoxid als Sonnenschutzmittel.

Überraschenderweise zeigen die gefundenen Formulierungen aus der Kombination der genannten Sonnenschutzmitteln mit dem Repellent sogar eine Erhöhung der Wirkdauer des Repellents gegenüber der entsprechenden Formulierung ohne UV-Filter. Die erfindungsgemäßen Formulierungen von UV-Filtern und Repellent zeigen weiterhin sehr geringe Hautpenetration des Repellents, einem der Hauptnachteile der bisherigen, meist auf dem Repellent DEET basierenden Kombinationsprodukte. Der Zusatz von UV-Filtern nach den erfindungsgemäßen Formulierungen führt überraschenderweise sogar zu einer Verringerung der Hautpenetration um den Faktor ca. 1,5 im Vergleich zur Formulierung ohne UV-Filter. Weiterhin konnte durch verschiedene Studien die Hautverträglichkeit sowie die Wasserresistenz der erfindungsgemäßen Formulierungen nachgewiesen werden.

Das Insektenrepellent 1-sek-Butyloxycarbonyl-2-(2-hydroxyethyl)-piperidin wird üblicherweise als Racemat, das unter dem Namen Saltidin^{®} erhältlich ist, eingesetzt. Alternativ können anstatt des Racemats auch die vier optischen Isomere von Saltidin^{®} in reiner oder in Kombination mehrerer Isomere als insektenrepellierende Komponente eingesetzt werden. Bevorzugt wird Saltidin^{®} eingesetzt.

Die in den erfindungsgemäßen Formulierungen eingesetzten Sonnenschutzmittel sind 2,2'-[6-(4-methoxyphenyl)-1,3,5-triazin-2,4-diyl]bis{5-[(2-ethylhexyl)oxy]phenol}, welches z.B. unter dem Handelsnamen Tinosorb S^{®} (BASF) erhältlich ist, und Zinkoxid oder Titandioxid , bevorzugt feinverteiltes Titandioxid , welches z.B.unter dem Handelnamen Tego Sun TDEC^{®} (Evonik Goldschmidt) erhältlich ist.

Die Formulierungen bieten breiten, lang anhaltenden Schutz vor UVA- und UVB-Strahlung. So kann z.B.eine erfindungsgemäße Formulierung , die 15% Saltidin^{®} enthält, einen Lichtschutzfaktor (SPF) im Bereich von 10 bis 15 aufweisen.

Die erfindungsgemäßen Formulierungen können gegebenenfalls weitere Wirkstoffe, Trägerstoffe, Lösungs-und/oder Dispergiermittel, oberflächenaktive Substanzen sowie weitere Additive wie z.B. Duftstoffe enthalten.

Die erfindungsgemäßen Formulierungen lassen sich in allen in der Kosmetik üblichen Darreichungsformen erstellen, beispielsweise in Form von Lösungen, Emulsionen, Gelen, Salben, Pasten, Cremes, Pulvern, Stiften, Sprays oder Aerosolen aus Sprühdosen.

Die erfindungsgemäßen Formulierungen enthalten üblicherweise 1-30% Saltidin^{®}, 0,1-20 % feinverteiltes Titandioxid (z.B.Tego Sun TDEC^{®}) und 0,1-10% 2,2'-[6-(4-methoxyphenyl)-1,3,5-triazin-2,4-diyl]bis{5-[(2-ethylhexyl)oxy]phenol} (z.B.Tinosorb S^{®}), wobei die Prozentangaben Gew% sind und sich immer auf die Gesamtformulierung (=100%) beziehen. Daraus ergibt sich ein Verhältnis von Saltidin^{®} zu Titandioxid zu (2,2'-[6-(4-methoxyphenyl)-1,3,5-triazin-2,4-diyl]bis{5-[(2-ethylhexyl)oxy]phenol}) von 1: 0,1:0,1 bis 1:20:30.

Desweiteren können noch Lösungsmittel und Dispergiermittel wie z.B. Wasser und PEG 400 enthalten sein.

Bevorzugt enthalten die erfindungsgemäßen Formulierungen 10-20% Saltidin^{®}, z.B. 15% Saltidin^{®}, 10-15 % feinverteiltes Titandioxid (z.B.Tego Sun TDEC^{®}) und 0,5- 5% 2,2'-[6-(4-methoxyphenyl)-1,3,5-triazin-2,4-diyl]bis{5-[(2-ethylhexyl)oxy]phenol} (z.B.Tinosorb S^{®}).

Die insekten- und milbenabwehrende Wirkung der erfindungsgemäßen Zusammensetzungen hält lange an. Sie können daher mit gutem Erfolg zur Abwehr von schädlichen oder lästigen, saugenden und beißenden Insekten und Milben verwendet werden.

Zu den saugenden Insekten gehören im wesentlichen die Stechmücken (z. B. Aedes-, Culex- und Anopheles-Arten), Schmetterlingsmücken (Phletotomen), Gnitzen (Culicoides-Arten), Kriebelmücken (Simulium-Arten), Stechfliegen (z. B. Stomoxys Calcitrans), Tsetse-Fliegen (Glossina-Arten), Bremsen (Tabanus-, Haematopota- und Chrysops-Arten), Stubenfliegen (z. B. Musca domestica und Fannia canicularis), Fleischfliegen (z. B. Sarcophaga carnaria), Myiasis erzeugende Fliegen (z. B. Lucilia couprina, Chrysomyia chloropyga, Hypoderma bovis, Hypoderma lineatum Dermatobia hominis, Oestrus ovis, Gasterophilus intestinalis, Cochliomyia hominovorax), Wanzen (z. B. Cimex lectularius, Rhodnius prolixus, Triatoma infestans), Läuse (z.B. Pediculus humanus, Haematipinus suis, Damalina ovis), Lausfliegen (z. B. Melaphagus orinus), Flöhe (z. B Pulex irritans, Cthenocephalides canis, Xenopsylla cheopsis) und Sandflöhe (z. B. Dermatophilus penetrans).

Zu den beißenden Insekten gehören im wesentlichen Schaben (z. B. Blattela germanica, Periplaneta americana, Blatta orientalis, Supella supellectilium), Käfer (z. B. Sitophilus granarius, Tenebrio molitor, Dermestes lardarius, Stegobium paniceum, Anobium puntactum, Hylotrupes bajulus), Termiten (z. B. Reticulitermes lucifugus) und Ameisen (z. B. Lasius niger).

Zu den Milben gehören Zecken (z. B. Ornithodorus Moubata, Ixodes ricinus, Boophilus microplus, Amblyomma hebreum) und Milben im engeren Sinne (z. B. Sarcoptes Scabiei, Dermanyssus gallinae).

Die vorliegende Erfindung betrifft somit auch die Verwendung der beschriebenen erfindungsgemäßen Zusammensetzungen zur Insekten- und Milbenabwehr. Ein weiterer Gegenstand der Erfindung ist daher die Verwendung einer erfindungsgemäßen insektenrepellierenden Formulierung eines Sonnenschutzmittels zum Schutz der menschlichen Haut vor UV-Sonnenlicht und Insektenstichen. Hierfür werden die erfindungsgemäßen Formulierungen auf die menschliche Haut aufgebracht.

Ein weiterer Gegenstand der Erfindung ist ein Verfahren zur Herstellung einer insektenrepellierenden Formulierung eines Sonnenschutzmittels, bei dem man das Insektenrepellent 1-sek-Butyloxycarbonyl-2-(2-hydroxyethyl)-piperidin und 2,2'-[6-(4-methoxyphenyl)-1,3,5-triazin-2,4-diyl]bis {5-[(2-ethylhexyl)oxy]phenol} und Zinkoxid oder Titandioxid als Sonnenschutzmittel gegebenenfalls mit Lösungs-und/oder Dispergiermitteln, Trägerstoffen und /oder oberflächenaktiven Substanzen und weiteren Wirkstoffen und/oder Additiven miteinander vermischt.

Die erfindungsgemäßen Formulierungen werden bevorzugt durch Vermischen des Insektenrepellents und der beiden Sonnenschutzmittel mit Lösungsmitteln, (z. B. Paraffine, Methanol, Ethanol, Isopropanol, Wasser), Trägerstoffen (z. B. Polyoxyethylen-Fettsäure-Ester, Polyoxyethylen-Fettalkohol-Ether, Alkylsulfonate, Arylsulfonate) und/oder oberflächenaktiven Substanzen und gegebenenfalls Additiven wie z.B. Duftstoffen hergestellt.

Bevorzugt werden die erfindungsgemäßen Formulierungen mit wässriger Lauge, insbesondere Natronlauge auf einen pH-Wert im Bereich zwischen 5,0 und 6,0 eingestellt. Dies entspricht in etwa dem pH-Wert der menschlichen Haut.

### Testmethoden

### Repellenttests:

Repellent-Effektivität von Formulierungen zur Anwendung gegen Moskitos am menschlichen Arm:
Die Insekten wurden als eine aktiv beißende Population (ca. 1000 Moskitos beiderlei Geschlechts) in Käfigen (90 cm lang, 30 cm breit, 40 cm hoch, Seitenwände aus Gaze) gehalten, der 2 leichte 5 Gewebeschleusen an der Vorderseite hat. Die Insekten sind ausschließlich mit Zuckerwasser gefüttert worden (10 % Dextropur). Das Alter der Insekten betrug mindestens 7 Tage, die Anzahl der Insekten wurde zwei mal pro Woche durch drei Tage alte ausgewachsene Insekten aufgefüllt.
Die Bissaktivitäten wurden während der Testperiode fortlaufend stündlich durch Aussetzen eines unbehandelten Arms an die Insekten überprüft (ein zusätzlicher interner Produktstandard wird von einem ausgesuchten Freiwilligen genutzt).

Die geringe elektrische Beleuchtung des Käfigs war aktiv von 6 Uhr morgens bis 6 Uhr abends, Licht von 6 Uhr abends bis 6 Uhr morgens. Die Temperatur betrug 25-27°C, die relative Luftfeuchtigkeit beträgt 50-70%.

Die Unterarme der Versuchspersonen wurden mit unparfürmierter Seife gewaschen, mit Wasser abgespült, dann mit einer Lösung aus 70 % Ethanol und 30 % Wasser abgespült und mit einem Handtuch getrocknet.

90cm² jedes Unterarms einer Versuchsperson wurden gleichmäßig mit 150 µl (oder 150 mg) des Testproduktes eingerieben. Sobald die Formulierung trocken war (nach ca. 5 Minuten) wurde eine Manschette mit einer Öffnung von 3,1-8 cm (25 cm²) so um den Arm gebunden, dass die Öffnung komplett über der behandelten Oberfläche lag. Die Ecken der Öffnung der Manschette waren ebenfalls mit dem Testmaterial eingerieben (200 µl) in einer Breite von 1 cm um Bisse an den Ecken zu vermeiden. Die Fläche oberhalb der Manschette wurde mit einem Tuch, das die Mücken nicht durchdringen können, geschützt. Hände wurden mit Latex-Handschuhen geschützt.

Beide Arme wurden in den Käfig durch die Gewebeschleuse eingeführt und die Anzahl der Bisse (und Landungen, falls nötig) pro Arm wurden in einer 3-minütigen Testperiode notiert. Der Test wurde stündlich bis zu 8 Stunden wiederholt oder vorher beendet, falls die Wirkung aussetzte (drei oder mehr Bisse innerhalb von 3 Minuten oder während 2 nacheinander folgenden Testsequenzen).

### Ermittlung des Sonnenschutzfaktors und Test auf Wasserresistenz:

Die Ermittlung des Sonnenschutzfaktors und die Wasserresistenz-Bestimmung wurden auf Grundlage der Internationalen-SPF-Testmethode 2006 und der Richtlinie zur Auswertung der Wasserresistenzbestimmung von Sonnenschutzprodukten (COLIPA 2005) an 12 Probanden durchgeführt. Zur Bestimmung des Sonnenschutzfaktors wurden den Probanden verschiedene Stellen des Rückens mit der Testformulierung bestrichen (36 cm² Hautfläche mit 2 mg/cm² Testformulierung) und anschließend mit einer 300W Xenon-Lampe in sechs verschiedenen Dosen bestrahlt. Die dabei resultierende Hautrötung diente als Kriterium zur Ermittlung des MED (Minimum erythema dose = minimale Hautrötungs-Dosis).

Der Sonnenschutzfaktor berechnet sich aus dem Quotienten von MEDᵤ (ungeschützte Haut) und MEDₚ (mit Testformulierung geschützte Haut). Der Gesamt-Sonnenschutzfaktor entspricht dem arithmetischen Mittel aller Probanden.

Die Wasserfestigkeit der Testformulierung wurde durch Vergleich des SPF vor und nach einem zweimaligen Aufenthalt der Probanden in einem Wasserbecken (je 20min) bestimmt.

### Test auf Hautverträglichkeit:

Die Hautverträglichkeit wurde nach der Methode von Walberg et al. mittels eines Patch-Testes unter Okklusion (Patch testing; in: Frosch P.J., Menné T., Lepoittevin J.-P. (Eds) Contact dermatitis (4th edition), Springer, Heidelberg, 366-386, 2006) an 50 Probanden durchgeführt. Dazu wurde ein Pflaster mit der Testformulierung getränkt und 24h bzw. 48h auf den Rücken des Probanden geklebt. Nach Entfernung des Pflasters wurde die Haut dermatologisch untersucht.

### Bestimmung der Hautpenetration:

Zur Bestimmung der Hautpenetration des Insektenrepellents wurde [¹⁴C]-markiertes Saltidin^{®} zur Herstellung der Sonnenschutzformulierung verwendet (Formulierung A). Zum Vergleich wurde die gleiche Formulierung ohne UV-Filter hergestellt und ebenfalls die Hautpenetration an isolierter menschlicher Haut gemessen. Dazu wurde eine definierte Menge der beiden Formulierungen aufgetragen (0,63-0,84 mg/cm²) und die Hautpenetration des radioaktiv markierten Saltidin^{®} bestimmt. Die Studie wurde nach den folgenden Richtlinien durchgeführt: OECD-guideline for the testing of chemicals, Skin absorption: in vitro method guideline 428 (2008); OECD environmental health and safety publication series on testing and assessment N° 28; Guidance document for the conduct on dermal absorption- Sanco/222/2000 rev. 7, 2004.

### Beispiele

### Formulierung A

Sonnenschutzemulsion mit 15% Insektenrepellent

**Tabelle 1: Zusammensetzung der Sonnenschutzemulsion A mit 15% Saltidin^{®}.**

| **Nr.** | **Inhaltsstoff** | **Gehalt [Gew-%]** |
|---|---|---|
| 1 | Saltidin^{®} | 15.0 |
| 2 | Glycerin | 5.0 |
| 3 | Ethanol abs. DAB | 8.0 |
| 4 | Carbomer (Carbopol Ultrez 10) | 0.2 |
| 5 | C₁₀-₃₀Alkylacrylat Crosspolymer (Pemulen TR1) | 0.1 |
| 6 | Polyethylenglycol 400 | 3.0 |
| 7 | Titandioxid (TegoCare TDEC sun) | 10.0 |
| 8 | (2,2'-[6-(4-methoxyphenyl)-1,3,5-triazin-2,4-diyl]bis{5-[(2-ethylhexyl)oxy]phenol}) (Tinosorb S^{®}) | 0.75 |
| 9 | Polyglyceryl-4-laurat (Tego Care LTP) | 3.0 |
| 10 | Duftstoff (Floral D 12889 G) | 0.5 |
| 11 | Wasser | 54.36 |
| 12 | Natronlauge (45%) | 0.09 |

### Herstellung:

Lösung 1: 1 u. 8 wurden bei 70°C-80°C gelöst und 15min gerührt.
Lösung 2: 4, 5, 7, 9 wurden bei Raumtemperatur gemischt und 15min gerührt.
Lösung 3: 2, 3, 6, 11 wurden bei Raumtemperatur gemischt und 15min gerührt.
Lösung 4: 3, 10 wurden bei Raumtemperatur gemischt.
Lösung 2 wurde zu Lösung 3 gegeben ohne Rühren und anschließend wurd homogenisiert.

Dann wurde die auf 50°C abgekühlte Lösung 1 bei Raumtemperatur unter Rühren zugetropft. Anschließend wurde homogenisiert. Danach wurde unter Rühren Lösung 4 zugetropft und homogenisiert.

Abschließend wurde der pH-Wert mit Natronlauge auf 5.0 - 6.0 eingestellt.

### Fomulierung B (höherer Sonnenschutzfaktor)

Sonnenschutzemulsion mit 15% Insektenrepellent

**Tabelle 2: Zusammensetzung der Sonnenschutzemulsion B mit 15% Saltidin^{®}.**

| **Nr.** | **Inhaltsstoff** | **Gehalt [Gew-%]** |
|---|---|---|
| 1 | Saltidin^{®} | 15.0 |
| 2 | Glycerin | 5.0 |
| 3 | Ethanol abs. DAB | 8.0 |
| 4 | Carbomer (Carbopol Ultrez 10) | 0.2 |
| 5 | C₁₀-₃₀Alkylacrylat Crosspolymer (Pemulen TR1) | 0.1 |
| 6 | Polyethylenglycol 400 | 3.0 |
| 7 | Titandioxid (TegoCare TDEC sun) | 12.0 |
| 8 | (2,2'-[6-(4-methoxyphenyl)-1,3,5-triazin-2,4-diyl]bis {5-[(2-ethylhexyl)oxy]phenol}) (Tinosorb S^{®}) | 2.0 |
| 9 | Polyglyceryl-4-laurat (Tego Care LTP) | 2.5 |
| 10 | C₁₂-₁₅Alkylbenzoat (Tegosoft TN) | 5.0 |
| 11 | Duftstoff (Floral D 12889 G) | 0.5 |
| 12 | Wasser | 46.61 |
| 13 | Natronlauge (45%) | 0.09 |

### Herstellung:

Lösung 1: 1, 8, 10 wurden bei 70°C-80°C gelöst und 15min gerührt.
Lösung 2: 4, 5, 7, 9 wurden bei Raumtemperatur gemischt und 15min gerührt.
Lösung 3: 2, 3, 6, 12 wurden bei Raumtemperatur gemischt und 15min gerührt.
Lösung 4: 3, 11 wurden bei Raumtemperatur gemischt.
Lösung 2 wurde zu Lösung 3 gegeben ohne Rühren und anschließend homogenisiert.

Dann wurde die auf 50°C abgekühlte Lösung 1 bei Raumtemperatur unter Rühren zugetropft. Anschließend wurde homogenisiert. Danach wurde unter Rühren Lösung 4 zugetropft und homogenisiert.

Abschließend wurde der pH-Wert mit Natronlauge auf 5.0 - 6.0 eingestellt.

### Ergebnisse

### Repellenttest

**Tabelle 3: Ergebnis des Repellenttest gegen Aedes aegypti am menschlichen Unterarm (Formulierung A)**

| Formulierung | Testperson/Wirkdauer [h] | | | | | Ø |
|---|---|---|---|---|---|---|
| | A | B | C | D | E | |
| mit Sonnenschutz | 7 | 7 | 7 | 9 | 9 | 7,8 |
| ohne Sonnenschutz | 6 | 6 | 5 | 8 | 10 | 7 |

Wie der Tabelle 3 zu entnehmen ist, ist die repellierende Wirkungsdauer mit den Sonnenschutzmitteln zumeist länger als ohne.

### Ermittlung des Sonnenschutzfaktors und Test auf Wasserresistenz:

Bei den Testformulierungen wurde ein SPF von 10 (Formulierung A) bzw. 15 (Formulierung B) ermittelt. Exemplarisch wurde an Formulierung A die Wasserresistenz getestet und als "wasserresistent" mit einer Wasserresistenz von 93% eingestuft.

### Test auf Hautverträglichkeit:

Exemplarisch wurde die Formulierung A dermatologisch untersucht und erhielt die Einstufung "hautverträglich".

### Hautpenetration

Exemplarisch wurde Formulierung A auf mögliche Hautpenetration des Repellents untersucht. Nach 24h wurde eine potentielle Absorption von Saltidin^{®} von 4,93% bestimmt, für die Vergleichs-Formulierung (ohne UV-Filter) wurde eine potentielle Absorption von 6,75% bestimmt.

## Patentansprüche

1. Insektenrepellierende Formulierung eines Sonnenschutzmittels, enthaltend das Insektenrepellent 1-sek-Butyloxycarbonyl-2-(2-hydroxyethyl)-piperidin und 2,2'-[6-(4-methoxyphenyl)-1,3,5-triazin-2,4-diyl]bis{5-[(2-ethylhexyl)oxy]phenol} und Titandioxid oder Zinkoxid als Sonnenschutzmittel.

2. Insektenrepellierende Formulierung eines Sonnenschutzmittels nach Anspruch 1, **dadurch gekennzeichnet, dass** das Insektenrepellent Saltidin^{®} ist.

3. Insektenrepellierende Formulierung eines Sonnenschutzmittels nach Anspruch 1 oder 2, **dadurch gekennzeichnet, dass** die Formulierung 1-30% Saltidin^{®}, 0,1-20 % feinverteiltes Titandioxid und 0,1-10% 2,2'-[6-(4-methoxyphenyl)-1,3,5-triazin-2,4-diyl]bis{5-[(2-ethylhexyl)oxy]phenol} enthält, wobei die Prozentangaben Gew% sind und sich immer auf die Gesamtformulierung beziehen.

4. Insektenrepellierende Formulierung eines Sonnenschutzmittels nach mindestens einem der Ansprüch 1 bis 3, **dadurch gekennzeichnet, dass** sie gegebenenfalls weitere Wirkstoffe, Trägerstoffe, Lösungs-und/oder Dispergiermittel, oberflächenaktive Substanzen sowie weitere Additive enthalten.

5. Verfahren zur Herstellung einer insektenrepellierenden Formulierung eines Sonnenschutzmittels, **dadurch gekennzeichnet, dass** man das Insektenrepellent 1-sek-Butyloxycarbonyl-2-(2-hydroxyethyl)-piperidin mit den Sonnenschutzmitten 2,2'-[6-(4-methoxyphenyl)-1,3,5-triazin-2,4-diyl]bis{5-[(2-ethylhexyl)oxy]phenol} und Titandioxid oder Zinkoxid miteinander vermischt.

6. Verfahren zur Herstellung einer insektenrepellierenden Formulierung eines Sonnenschutzmittels nach Anspruch 5, **dadurch gekennzeichnet, dass** man den pH-Wert der Formulierung mit einer wässrigen Lauge auf einen Wert im Bereich zwischen 5,0 und 6,0 einstellt.

7. Verwendung einer insektenrepellierenden Formulierung eines Sonnenschutzmittels gemäß einen der Ansprüche 1 bis 4 zum Schutz der menschlichen Haut vor UV-Sonnenlicht und Insektenstichen.
